# EUROPEAN PATENT SPECIFICATION

(11) **EP 0 372 636 B1**
(45) Date of publication and mention of the grant of the patent: **09.02.1994**
(21) Application number: 89203046.1
(22) Date of filing: 30.11.1989
(51) Int. Cl.: G01N 3/18

(54) **A method and cell for freezing formation layer sample tests**
Verfahren und Zelle zur Gefrierprüfung von Proben geologischer Schichten
Méthode et cellule d'essais de congélation sur un échantillon d'une couche géologique

(30) Priority: 02.12.1988 GB 8828205
(43) Date of publication of application: 13.06.1990
(73) Proprietor: SHELL INTERNATIONALE RESEARCH MAATSCHAPPIJ B.V., 2596 HR Den Haag (NL)
(72) Inventor: Zorn, Nils Friedrich, NL-2600 AB Delft (NL); Horvat, Endre, NL-2596 HR The Hague (NL); Unsworth, John Francis, Ince, Chester, Cheshire CH1 3SH (GB)

(56) References cited:
- SOVIET INVENTIONS ILLUSTRATED, week 8702, 14th January 1987, vedh. section S03, accession no. 87-013705/02, Derwent Publications Ltd, London, GB; & SU-A-1233 004 (VEDENEEV HYDROTECH.) 23-05-1986
- SOVIET INVENTIONS ILLUSTRATED, week 8539, 8th November 1985, Moen. section S03, accession no. 85-241845/39, Derwent Publications Ltd, London, GB; & SU-A-1146 573 (MOX. ENG. CONS. INST.) 23-03-1985
- SOVIET INVENTIONS ILLUSTRATED, week C39, 8th November 1980, shik. section S03, accession no. J4165 C/39, Derwent Publications Ltd, London, GB; & SU-A-714 281 (SHIKHOV V.N.) 05-02-1980
- OIL & GAS JOURNAL, vol. 81, no. 10, March 1983, pages 118-120; A. BOULANGER et al.: "Pilot project shows liquefied gas is stored successfully at low temperature in clay"
- REVIEW OF SCIENTIFIC INSTRUMENTS, vol. 53, no. 3, August 1982, pages 1246-1254; D. TRIMMER: "Laboratory measurements of ultralow permeability of geologic materials"
- INSTRUMENT PRACTICE, vol. 25, no. 11, November 1971, pages 636-638; P.K. SAGE: "Instruments and tests in soil mechanics"

## Description

The present invention relates to a method and cell for determining temperature dependant stress-strain characteristics of a formation layer sample during a freezing process.

The present invention particularly relates to a method and cell wherein a freezing process in a formation layer is simulated such as when a cavern therein is filled with a liquified gas. More specifically caverns in soil and rock layers are intended for storage of liquified gases such as propane, ethylene and liquified natural gas, LNG, with respective boiling points of -42 °C, -104 °C and -162 °C.

As known from A. Boulanger et al. "Pilot project shows liquified gas is stored successfully at low temperature in clay", Oil & Gas Journal, vol. 81, No. 10, March 1983, in this field of application pilot tests have been carried out in Belgian formation layers, so-called Boom-clay layers. In said tests a pilot cavity was created at 23 m beneath the earth surface. Said cavity had a diameter of 3 m and a length of 30 m. The lowering of the temperature was accomplished by spraying liquid nitrogen into said cavity. During the entire 10 week test period the behaviour of the cavity and the surrounding area was monitored by more than three hundred measurement points. Among the different types of sensors thermocouples, vibrating wire extensometers, clinometers and bore-hole extensometers were used. After said 10 week period the cavity was found to be perfectly sealed and surrounded by approximately 6 m of frozen clay which was not fractured in any way. Moreover, a laboratory test programme on samples from the above formation layer revealed some "in situ" layer characteristics i.e. the thermal diffusivity and the compressive strength at various temperatures. The said characteristics were compared with and were said to be in good agreement with the above pilot test results.

However, it is very expensive to carry out such pilot tests in different formation layers, particularly when investigating rock formation layers. Moreover, soil and/or rock properties are in terms of material science not well known, since they are influenced by numerous parameters such as the ratio of mineral:water:void content, the grain size distribution of the mineral content, the minerals and composition of the mixture, the stress boundary conditions, the permeability or drainage conditions, the strain rate dependance and strain history and the effective particle stress level.

As can be read from P. Sage, "Instruments and Tests in Soil Mechanics", Instrument Practice, vol. 25, No. 11, November 1971, specifically in soil mechanics this has led to the development of triaxial soil testing techniques, in which a generally cylindrical sample is exposed to representative boundary conditions with respect to horizontal and vertical stresses and drainage conditions (Due to the fact that samples are retained from bore holes, or in soft soil using a continuous core sampler, the diameters are in the range of 60 to 100 mm). Under ideal conditions the tests are then performed on undisturbed samples of the soil under conditions that represent characteristic underground conditions for the loading of which the effect must be studied.

For samples taken at larger depths or from overconsolidated clay layers the test procedure, prior to the test itself, requires readaption to the representative conditions since these cannot be maintained throughout the handling of a sample. This process is referred to as consolidation, and can be explained as reachieving the conditions as in situ with respect to the magnitude and ratio of vertical (overburden) and horizontal (confining) pressure and the original corresponding porewater pressure and content.

In Sovjet Inventions Illustrated, week 8702, January 14, 1987, accession No. 87-013705/02, Derwent Publications, London, a soil test device for determination of water permeability and filtration capacity of a soil sample is shown. In order to start from well defined conditions said soil sample is frozen thereby locking the water within the sample. Subsequent thawing the sample by means of a pressurized water stream allows the above determination. In no way cooling the sample from an inner conduit to the outer sample parts can be seen from or is suggested in this document.

From Sovjet Inventions Illustrated, week 8539, November 8, 1985, accession No. 85-241845/39, Derwent Publications, London, a ground soil stability measuring device is known. In particular a pressure control system is shown for controlling vertical and horizontal pressures upon a soil sample. Via an outlet conduit in the upper part of the device filtration flow is measured. No further temperature characteristics of such a sample are addressed.

In such above triaxial tests extended temperature behaviour of formation layer samples during a freezing process is unknown up to now. In particular it is of importance to test samples under drained or undrained conditions. In the first case porewater can flow in and out of the sample during the test, while the volume flow is measured. In the second case the sample is sealed after consolidation and the porewater flow is bounded within the sample during the test. In this case the porewater pressure can be measured.

Thus, it is an object of the invention to improve such tests in which soil and/or rock parameters can be measured during a freezing process thereby reaching deep cooled circumstances, so-called cryogenic circumstances.

It is a further object of the invention to measure soil and/or rock parameters which are highly representative for the above-mentioned characteristic underground conditions.

It is another object of the invention to obtain reproducible results from which storage criteria can be derived allowing a reliable underground storage of liquified gases such as LNG.

The invention therefore provides a method for determining temperature dependant stress-strain characteristics of a formation layer sample said method comprising the following steps:
a) simulating characteristic underground conditions for said sample,
b) creating an axial central hole in said sample which functions as a coolant conduit,
c) arranging said sample in a triaxial cell in which said sample is loaded to predetermined test pressures,
d) cooling said sample during a freezing process outwardly from said central hole by circulating a coolant through the central hole while exerting said test pressures, said coolant being supplied into and discharged from said hole through conduit means, and
e) monitoring during said freezing process temperature dependant sample parameters with which said stress-strain characteristics are determined.

Alternatively in the above method step b) is effected before step a).

The invention further provides a method wherein said test pressures comprise simulating in situ condition pressures.

In a further embodiment of the above method said axial central hole is created continuously throughout said sample.

Advantageously for said sample horizontal and vertical deformation, the transport and volume of unbound water, and the pressure and volume of unbound water in an undrained sample are measured, and cracking investigation is performed during such a freezing process. The mentioned parameters can be measured separately or in combination as desired. Furthermore, a controlled coolant supplying rate is provided.

The invention further provides a triaxial cell for determining temperature dependant stress-strain characteristics of a formation layer sample arranged in said cell, said cell comprising:
- means for loading said sample to predetermined test pressures,
- conduit means for supplying, circulating and discharging a coolant for cooling said sample outwardly from a central hole during a freezing process,
- means for measuring during said freezing process temperature dependant sample parameters with which said stress-strain characteristics are determined, and
- means for thermally isolating said sample during said freezing process.

The cell in accordance with the invention further comprises a bellow means supplied with a pressurizing fluid for vertically loading said sample to said predetermined test pressures, laser optical means for measuring horizontal and vertical deformations of said sample and transport means connected with said sample for measuring the transport and volume of unbound water.

The invention will now be described by way of example in more detail with reference to the accompanying drawings, wherein
- Fig. 1 is a plot which schematically shows the behaviour of the proportional change of volume for a clay sample and a rock sample when lowering the temperature,
- Fig. 2 shows schematically an embodiment of a triaxial cell in accordance with the invention,
- Fig. 3A and 3B are graphs, giving curves as measured for a clay sample in accordance with the invention, and
- Fig. 4A and 4B are graphs, giving curves as measured for a rock sample in accordance with the invention.

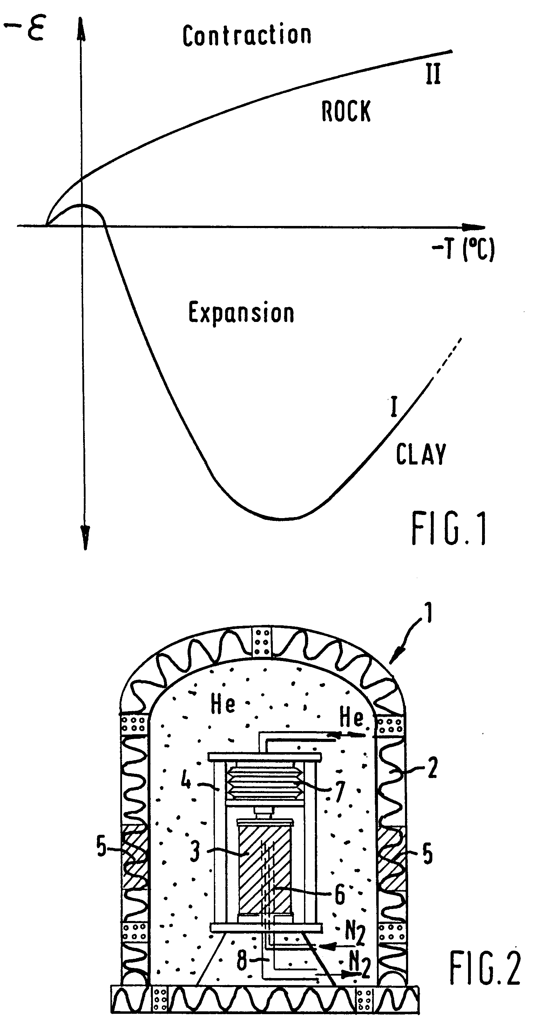

Referring to fig. 1 a plot schematically gives the behaviour of the proportial change of volume, or volumetric change, for a clay sample I and a rock sample II as function of the temperature.

The clay curve I first shows a contraction due to the presence of mineral compounds in the clay sample whereafter an expansion occurs caused by the presence of water within such a clay sample. When the temperature is lowered substantially again a contraction mechanism will dominate the sample behaviour. It will be clear that such a minimum as shown in the clay curve I will be an important criterion for the utility of such formation layers in case liquified gases have to be stored therein. Possible cracking phenomena will be revealed thereby.

On the contrary the rock curve II only shows a contraction because hardly any free water will be present in such rock samples.

Referring to fig. 2 an embodiment of a triaxial cell 1 in accordance with the present invention has been shown. Particularly deep cooling of such a formation layer sample in a cryogenic test is required when LNG storage has to be simulated. For that purpose the cell 1 has vacuum isolated walls 2. A formation layer sample 3, having for example a cylinder form, is arranged within said cell 1.

The sample 3 is placed within a load frame 4 to achieve and/or to maintain predetermined test pressures. More in detail one has to distinguish separately the vertical load conditions, or overburden pressures, and the horizontal conditions, or confining pressures. In such tests various levels and combinations of overburden and confining pressures are exerted onto such a sample including in situ condition pressures which correspond with characteristic underground conditions. For clay samples in general the overburden pressures are about a factor two greater than the confining pressures. On the contrary for rock samples vertical and horizontal pressures are more or less equal.

To achieve said pressure conditions a sample 3 is arranged in an overpressurized environment, for example by means of an overpressurizing fluid such as silicon oil. When deep cooled conditions are desired helium gas, as shown in fig. 2, is used advantageously. Then for a clay sample, the load frame 4 can comprise a bellow means 7 as a load frame, supplied with additional helium gas in order to obtain the required vertical pressure. Generally a sealing against penetration of pressurized fluid will be necessary for both clay and rock samples. Such sealing can be achieved for example by means of a fluid tight sleeve covering the whole free sample surface, i.e. the side and top surfaces of the sample 3 as shown in fig. 2.

In order to determine temperature dependant stress-strain characteristics of such a formation layer sample 3 during a freezing process monitoring of parameters has to be done. Relative thereto one has to measure, separately or in combination, vertical and horizontal deformation, transport and volume of unbound water, pressure and mass of unbound water within undrained samples and temperature gradients within such samples. Also crack inspection has to be performed.

Implementing the above measurements can be effected on various ways. Conventionally vertical and horizontal deformations are measured respectively by means of displacement transducers and gap sensors. However, in deep cooling circumstances the said measuring equipment may give inaccurate and unreliable data. Therefore an other type of measuring has to be used, for example the optical type. Also linear voltage displacement transducers (LVDT) may be used. As can be seen in fig. 2 looking glasses 5 are provided in the wall of the triaxial cell 1 to perform the said optical measurements.

When simulating said underground conditions a pore water flow can occur. More specifically the gradient of the pore water flow presents vital information about the sample freezing conditions. Thereby the transport and volume of unbound water will be measured.

Therefore a transport means, such as a drain pipe, (which is not shown in fig. 2 for reason of clarity) is arranged within the sample 3 as a conduit to transport the unbound water from and into the sample. Advantageously the transport means is connected with a water reservoir.

On the contrary an undrained sample is used when the pressure of unbound water within said sample has to be measured. Therefore a pressure detector (not shown in fig. 2 for reason of clarity) has to be connected with said sample.

In addition to measuring of transport volume and pressure of unbound water as mentioned above also the volume of the unbound water within said sample can be monitored with aid of advanced technology like nuclear magnetic resonance (NMR). Thereby two NMR techniques, i.e. the continuous wave techniques and the pulsed NMR technique, can complement each other.

Furthermore, temperature measuring means (not shown for reason of clarity) are arranged within the sample 3. Advantageously a plurality of temperature sensors, for example a number of four sensors, is inserted into the sample from the bottom, the sensors being positioned at different distances from the central axis of the sample. Then the temperature gradient from the inner region to the outside region can be monitored, thereby simulating in situ monitoring when storing liquefied gases in a cavern beneath the earth surface.

To achieve the required cryogenic temperatures in such samples a coolant is introduced into the sample 3 through a hole 6 created in the sample. Advantageously said hole 6 is created along the central axis of the sample 3. Furthermore said hole is bored not entirely throughout said sample, thus simulating a cavernlike hole. A conduit means 8 communicates with the inner region of the sample, supplying and discharging the coolant into and from said region. It has been proven advantageous to supply said coolant in a controlled way in which different coolant supply rates can be obtained.

Alternatively the central hole 6 is created continuously throughout the sample. Then a radially symmetric temperature loading of the sample is achieved.

Advantageously the coolant used in simulating cavern test procedures is nitrogen vapour or liquid nitrogen, the latter having a boiling temperature of -196 °C. Therewith a freezing process from ambient temperature to the boiling temperature of -162 °C of liquefied natural gas can be simulated suitably. In a complementary way after the above cooling a controlled warming-up can be accomplished. Both temperature courses will give specific parameter informations.

The temperature measurements can be performed by standard type temperature sensors. For example Pt-100 sensors will be used during such freezing periods.

Last mentioned, but not least of interest, crack investigation has to be performed. It appeares advantageous to use acoustic emission measurements to reveal possible cracks. Just like in the seismic field cracks will reflect (and also diffract) acoustical energy. Accurate processing of data so obtained will be accomplished in automated procedures performed by computer devices. Also crack inspection by means of video imaging accomplished with aid of an endoscope device can be employed.

It will be clear to those skilled in the art that measurements of the above type will provide a lot of information about the stress-strain characteristics of such formation layers. Thereby elastic and plastic properties like Young's modulus, Poisson ratio and yield strength can be calculated. Also permeability and porosity can be determined.

To further elucidate the above method and corresponding test procedures two examples will be shown.

### EXAMPLE 1

In example 1 test results relative to an artificial clay sample are disclosed. Some properties as determined for said clay sample are shown in Table 1.

**Table 1**

| | |
|---|---|
| density (dry) | 1755 ± 10 kg/m³ |
| water content | 18.9% (weight % relative to dry sample) |
| grain size distribution | 49.5% < 2 µm; 69% < 16 µm; |
| (weight %) | 85.0% < 63 µm |
| plastic limit | 18.5% |
| liquid limit | 38.1% |
| plasticity index | 19.6% |
| permeability | 1.0x10⁻¹⁰ m/s at 5 bar |
| porosity | 33.3% |
| mineral content (weight %) | quartz 50%, kaolinite 25%, illite 20%, chlorite 5% |

The sample was adopted (consolidated) to the mechanical boundary conditions, isotropic pressure of 5 bar for 17 hours, a period after which the gradients in volume change and porewater flow were negligible, and inserted into the cell for a drained triaxial test at constant stress level. The cooling rate chosen was linear at 20 °C per hour, measured at the temperature sensor nearest to the central cooling hole and the minimum temperature at this sensor was set to -50 °C.

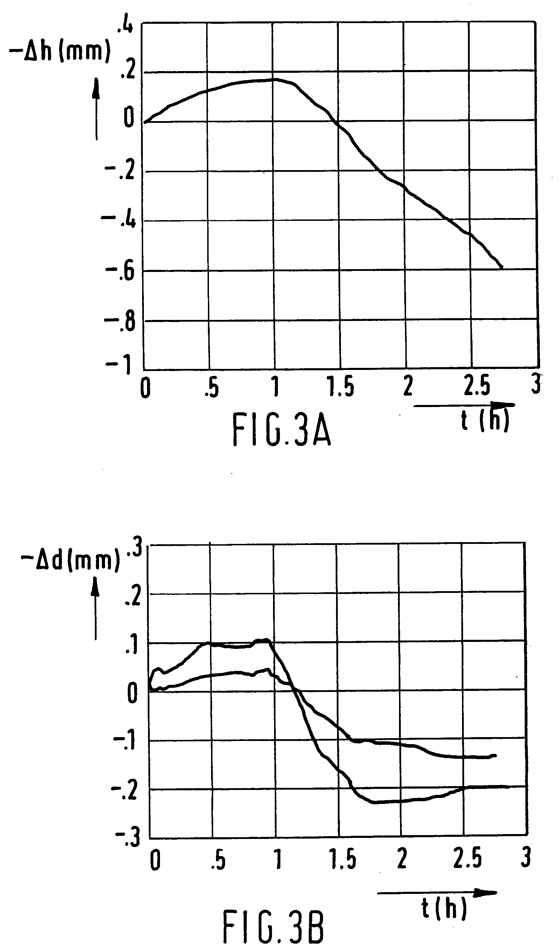

The results of the deformation measurements as recorded in an investigation run are presented in two graphs, shown in fig. 3A and fig. 3B. Fig. 3A shows the change in height as function of time the most integral measurement since it is averaged over the total height of the sample. Conventionally the contraction region is situated above the horizontal axis (see also fig. 1). Therefore the change of heigth (and also diameter) is indicated as -Δh(and -Δd). Furthermore it is noticed that because of the linear cooling process as stated above the shown time dependant courses are similar to temperature dependant courses such as explained relative to fig. 1.

It can be distinguished that the axial component contributing to the volumetric strain first shows a decrease and after 1.5 hours, corresponding to an average sample temperature of ± -5 °C, reaches its original size and then shows an increase.

The second deformation measurement is the change of diameter measured at two levels of the sample, generally ± 1/3 and ± 2/3 of the total height with three gap sensors each. The resulting value represents the diameter as shown in fig. 3B shows very similar characteristics as the change in height. The diameters decrease in the first stage of the loading and then increase. The initial diameter however is reached slightly earlier than the initial height. The difference in the amplitude of change in diameter for the two levels can be assigned to a temperature gradient in axial direction of the sample.

Deriving the volumetric strain from the product of the average change in the cross section derived from diameter changes and the change in height related to the original volume would thus result in a curve qualitatively following curve I as presented in fig. 1.

### EXAMPLE 2

In example 2 test results relative to a dry granite rock sample are disclosed. The boundary conditions and geometrical dimensions were kept the same as for the clay samples, and with exception of the consolidation phase which does not apply for granite (only the elastic deformation due to the loading must be considered), the same test and measuring procedure was followed.

The effected cooling was also a linear process. However, a temperature discontinuity (not shown in a separate graph for reason of simplicity) in the final condition occurred. A preliminary explanation of this is that local micro cracking provided short cut cooling channels between the temperature sensors.

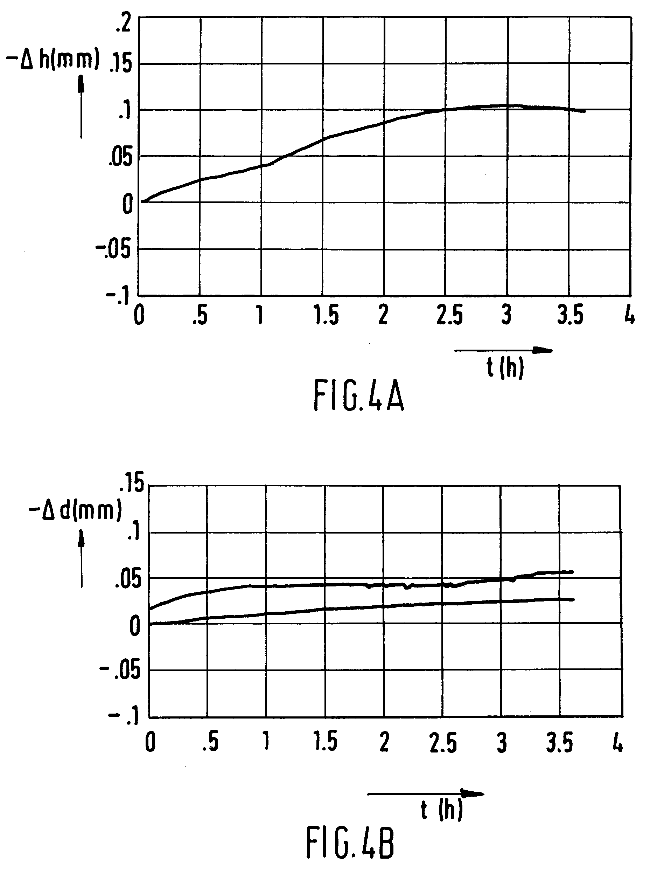

The deformation measurements presented in figures 4A and 4B can be described very briefly, since they require less interpretation than clay tests. The granite rock shows a steady contraction as reaction to the cooling. The derived volumetric strain thus would confirm the predictions.

## Claims

1. A method for determining temperature dependant stress-strain characteristics of a formation layer sample, said method comprising the following steps:
a) simulating characteristic underground conditions for said sample,
b) creating an axial central hole (6) in said sample (3) which functions as a coolant conduit,
c) arranging said sample (3) in a triaxial cell (1) in which said sample (3) is loaded to predetermined test pressures,
d) cooling said sample (3) during a freezing process outwardly from said central hole (6) by circulating a coolant through the central hole while exerting said test pressures, said coolant being supplied into and discharged from said hole (6) through conduit means (8), and
e) monitoring during said freezing process temperature dependant sample parameters with which said stress-strain characteristics are determined.

2. The method as claimed in claim 1, wherein step b) is effected before step a).

3. The method as claimed in claim 1 or 2, wherein said test pressures are in situ condition pressures.

4. The method as claimed in claim 2 or 3, wherein said axial central hole (6) is created continuously throughout said sample (3).

5. The method as claimed in any one of the claims 1-4, wherein the coolant is liquid nitrogen.

6. The method as claimed in any one of the claims 1-5, wherein horizontal and vertical deformation of said sample (3) are measured.

7. The method as claimed in any one of the claims 1-6, wherein the transport and volume of unbound water are measured.

8. The method as claimed in any one of the claims 1-6, wherein the pressure of unbound water contained in an undrained sample is measured.

9. The method as claimed in any one of the claims 1-8, wherein the volume of unbound water within said sample is measured by using a nuclear magnetic resonance (NMR) technique.

10. The method as claimed in claim 9, wherein said NMR-technique is a pulsed NMR-technique.

11. The method as claimed in claim 9, wherein said NMR-technique is a continuous wave technique.

12. The method as claimed in any one of the claims 1-9, wherein a crack inspection is performed during said freezing process.

13. The method as claimed in claim 12, wherein said crack inspection is accomplished using an acoustical investigation technique.

14. The method as claimed in claim 12, wherein said crack inspection is accomplished using an endoscope technique.

15. The method as claimed in any one of the claims 1-14, wherein a controlled coolant supplying rate is provided.

16. A triaxial cell (1) for determining temperature dependant stress-strain characteristics of a formation layer sample (3) arranged in said cell (1), said cell (1) comprising:
- means (4,7) for loading said sample (3) to predetermined test pressures,
- conduit means (8) for supplying, circulating and discharging a coolant for cooling said sample (3) outwardly from a central hole (6) during a freezing process,
- means for measuring during said freezing process temperature dependant sample (3) parameters with which said stress-strain characteristics are determined, and
- means (2) for thermally isolating said sample (3) during said freezing process.

17. The cell as claimed in claim 16 further comprising a bellow means supplied with a pressurizing fluid for vertically loading said sample (3) to said predetermined test pressures.

18. The cell as claimed in claim 16, further comprising laser optical means for measuring horizontal and vertical deformations of said sample (3).

19. The cell as claimed in claim 16, further comprising transport means connected with said sample (3) for measuring the transport volume of unbound water.

20. Use of the method as claimed in any one of the claims 1-15 on samples (3) taken from formation layers.

## Patentansprüche

1. Eine Methode zum Bestimmen der temperaturabhängigen Spannungs-Dehnungs-Charakteristika einer Gebirgsprobe, welche Methode die folgenden Maßnahmen umfaßt:
a) Simulieren von charakteristischen Bedingungen im Untergrund für besagte Probe,
b) Ausbilden einer achsialen zentralen Aushöhlung (6) in besagter Probe (3), welche als Leitung für ein Kühlmittel dient,
c) Anordnen besagter Probe (3) in einer dreiachsigen Zelle (1), in welcher besagte Probe (3) bis zu vorbestimmten Versuchsdrucken druckbelastet wird,
d) Abkühlen besagter Probe (3) während eines Ausfrierverfahrens von besagter Aushöhlung (6) aus von innen nach außen, indem man ein Kühlmittel durch die achsiale zentrale Aushöhlung zirkulieren läßt, während gleichzeitig besagte Versuchsdrucke zur Einwirkung gebracht werden, wobei besagtes Kühlmittel über Leitungen (8) besagter Aushöhlung (6) zugeführt und daraus wieder abgezogen wird, und
e) Aufzeichnen von temperaturabhängigen Probeparametern während besagten Ausfrierverfahrens, mit denen besagte Spannungs-Dehnungs-Charakteristika bestimmt werden.

2. Eine Methode wie in Anspruch 1 beansprucht, bei der die Maßnahme b) vor der Maßnahme a) durchgeführt wird.

3. Eine Methode wie in Anspruch 1 oder 2 beansprucht, in welcher besagte Versuchsdrucke den vor Ort anzutreffenden Drucken entsprechen.

4. Eine Methode wie in Anspruch 2 oder 3 beansprucht, in welcher die besagte achsiale zentrale Aushöhlung (6) sich kontinuierlich durch die ganze Probe (3) erstreckend ausgebildet wird.

5. Eine Methode wie in Anspruch 1 bis 4 beansprucht, in welcher das Kühlmittel flüssiger Stickstoff ist.

6. Eine Methode wie in irgendeinem der Ansprüche 1 bis 5 beansprucht, in welcher die horizontale und die vertikale Deformation besagter Probe (3) gemessen werden.

7. Eine Methode wie in irgendeinem der Ansprüche 1 bis 6 beansprucht, in welcher der Transport und das Volumen von nicht gebundenem Wasser gemessen werden.

8. Eine Methode wie in irgendeinem der Ansprüche 1 bis 6 beansprucht, in welcher der Druck von nicht gebundenem Wasser in einer nicht entwässerten Probe gemessen wird.

9. Eine Methode wie in irgendeinem der Ansprüche 1 bis 8 beansprucht, in welcher das Volumen an nicht gebundenem Wasser in der Probe unter Verwendung einer kernmagnetischen Resonanztechnik (NMR) gemessen wird.

10. Eine Methode wie in Anspruch 9 beansprucht, in welcher es sich bei besagter NMR-Technik um eine pulsierende NMR-Technik handelt.

11. Eine Methode wie in Anspruch 9 beansprucht, in welcher es sich bei besagter NMR-Technik um eine kontinuierliche Wellentechnik handelt.

12. Eine Methode wie in irgendeinem der Ansprüche 1 bis 9 beansprucht, in welcher die Untersuchung auf Rißbildung während des besagten Ausfrierverfahrens durchgeführt wird.

13. Eine Methode wie in Anspruch 12 beansprucht, in welcher besagte Untersuchung auf Rißbildung mittels einer akustischen Untersuchungstechnik durchgeführt wird.

14. Eine Methode wie in Anspruch 12 beansprucht, in welcher besagte Untersuchung auf Rißbildung mittels einer Endoskoptechnik durchgeführt wird.

15. Eine Methode wie in irgendeinem der Ansprüche 1 bis 14 beansprucht, in welcher für eine kontrollierte Zuflußgeschwindigkeit des Kühlmittels gesorgt wird.

16. Eine dreiachsige Zelle (1) zur Bestimmung der temperaturabhängigen Spannungs-Dehnungs-Charakteristika einer Gesteinsprobe (3), welche in besagter Zelle (1) angeordnet ist, wobei die besagte Zelle (1) die folgenden Mittel umfaßt:
- Mittel (4,7), um besagte Probe (3) bis zu vorbestimmten Versuchsdrucken druckzubeladen,
- Leitungsmittel (8), um ein Kühlmittel zuzuführen, zirkulieren zu lassen und abzuführen zwecks Kühlung besagter Probe (3) von einer zentralen Aushöhlung (6) aus nach außen während eines Ausfrierverfahrens,
- Mittel zum Messen der temperaturabhängigen Parameter der Probe (3) während des Ausfrierverfahrens, mit welchen Parametern besagte Spannungs-Dehnungs-Charakteristika bestimmt werden, und
- Mittel (2) zur thermischen Isolierung besagter Probe (3) während des besagten Ausfrierverfahrens.

17. Die Zelle wie in Anspruch 16 beansprucht, welche außerdem ein blasebalgartiges Mittel umfaßt, welchem ein unter Druck stehendes Fluid zugeführt wird zwecks vertikaler Druckbelastung besagter Probe (3) bis auf die besagten vorbestimmten Versuchsdrucke.

18. Die Zelle wie in Anspruch 16 beansprucht, welche weiterhin laseroptische Mittel zur Messung von horiziontalen und vertikalen Deformationen besagter Probe (3) umfaßt.

19. Die Zelle wie in Anspruch 16 beansprucht, welche weiterhin Transportmittel umfaßt, welche mit besagter Probe (3) zwecks Messung des Transportvolumens von nicht gebundenem Wasser in Verbindung stehen.

20. Verwendung der Methode wie in irgendeinem der Ansprüche 1 bis 15 beansprucht, für Proben (3), die aus Gebirgsschichten entnomen wurden.

## Revendications

1. Procédé de détermination des caractéristiques de contrainte-déformation en fonction de la température d'un échantillon d'une couche d'une formation, ledit procédé comprenant les étapes suivantes :
a) simulation des conditions caractéristiques en sous-sol dudit échantillon,
b) création dans ledit échantillon (3) d'un trou axial central (6) qui assume la fonction d'un conduit d'agent réfrigérant,
c) mise en place dudit échantillon (3) dans une cellule triaxiale (1) dans laquelle ledit échantillon (3) est soumis à des pressions d'essai prédéterminés,
d) refroidissement dudit échantillon (3) pendant un processus de réfrigération vers l'extérieur dudit trou central (6) par mise en circulation d'un agent réfrigérant dans le trou central pendant que sont exercées lesdites pressions d'essai, ledit agent réfrigérant étant introduit dans et déchargé dudit trou (6) par un conduit (8) et
e) contrôle, pendant ledit processus de réfrigération, de paramètres de l'échantillon qui sont fonction de la température et à l'aide desquels lesdites caractéristiques de contrainte-déformation sont déterminées.

2. Procédé selon la revendication 1, dans lequel l'étape b) est effectuée avant l'étape a).

3. Procédé selon la revendication 1 ou 2, dans lequel lesdites pressions d'essai sont les conditions de pression in situ.

4. Procédé selon la revendication 2 ou 3, dans lequel ledit trou central axial (6) est créé de part en part dans ledit échantillon (3).

5. Procédé selon l'une quelconque des revendications 1 à 4, dans lequel l'agent réfrigérant est l'azote liquide.

6. Procédé selon l'une quelconque des revendications 1 à 5, dans lequel les déformations horizontale et verticale dudit échantillon sont mesurées.

7. Procédé selon l'une quelconque des revendications 1 à 6, dans lequel le transport et le volume d'eau non liée sont mesurés.

8. Procédé selon l'une quelconque des revendications 1 à 6, dans lequel la pression d'eau non liée que contient un échantillon non asséché est mesurée.

9. Procédé selon l'une quelconque des revendications 1 à 8, suivant lequel le volume d'eau non liée se trouvant dans ledit échantillon est mesuré par une technique de résonnance magnétique nucléaire (RMN).

10. Procédé selon la revendication 9, dans lequel ladite technique de RMN est une technique de RMN pulsée.

11. Procédé selon la revendication 9, dans lequel ladite technique de RMN est une technique à onde continue.

12. Procédé selon l'une quelconque des revendications 1 à 9, suivant lequel une recherche de fissures est effectuée pendant le processus de réfrigération.

13. Procédé selon la revendication 12, suivant lequel ladite recherche de fissures est effectuée à l'aide d'une technique d'investigation acoustique.

14. Procédé selon la revendication 12, suivant lequel ladite recherche de fissures est effectuée à l'aide d'une technique endoscopique.

15. Procédé selon l'une quelconque des revendications 1 à 14, suivant lequel le débit d'alimentation en agent réfrigérant est commandé.

16. Cellule triaxiale (1) de détermination de caractéristiques de contrainte-déformation en fonction de la température d'un échantillon (3) d'une couche d'une formation qui est placée dans ladite cellule (1), ladite cellule (1) comprenant :
- des moyens (4, 7) pour soumettre ledit échantillon (3) à des pressions prédéterminées d'essai,
- un conduit (8) d'arrivée, de circulation et de décharge d'un agent réfrigérant destiné à refroidir ledit échantillon (3) d'un trou central (6) vers l'extérieur pendant un processus de réfrigération,
- des moyens de mesure, pendant ledit processus de réfrigération, de paramètres de l'échantillon (3) qui sont fonction de la température et à l'aide desquels lesdites caractéristiques de contrainte-déformation sont déterminées et
- des moyens (2) d'isolation thermique dudit échantillon (3) pendant ledit processus de réfrigération.

17. Cellule selon la revendication 16, comprenant par ailleurs un soufflet alimenté en fluide comprimé pour exercer une charge verticale sur ledit échantillon (3) auxdites pressions prédéterminées d'essai.

18. Cellule selon la revendication 16, comprenant par ailleurs des moyens optiques à laser de mesure des déformations horizontale et verticale dudit échantillon (3).

19. Cellule selon la revendication 16, comprenant par ailleurs des moyens de transport raccordés audit échantillon ( 3) pour la mesure du volume transporté d'eau non liée.

20. Mise en oeuvre du procédé selon l'une quelconque des revendications 1 à 15 sur des échantillons (3) prélevés sur des couches de formations.
